# EUROPEAN PATENT SPECIFICATION

(11) **EP 2 713 931 B1**
(45) Date of publication and mention of the grant of the patent: **26.12.2018**
(21) Application number: 12793169.9
(22) Date of filing: 01.06.2012
(51) Int. Cl.: A61B 34/00, B25J 13/06, B25J 9/06, A61B 17/29

(54) **ROBOTIC SYSTEM USER INTERFACES**
ROBOTERSYSTEM-BENUTZERSCHNITTSTELLEN
INTERFACES UTILISATEUR DE SYSTÈME ROBOTIQUE

(30) Priority: 02.06.2011 US 201161492578 P; 13.09.2011 US 201161534032 P; 21.12.2011 US 201161578582 P
(43) Date of publication of application: 09.04.2014
(73) Proprietor: Medrobotics Corporation, Raynham, MA 02767 (US)
(72) Inventor: FLAHERTY, J. Christopher, Auburndale, Florida 33823 (US); ZUBIATE, Brett, Pittsburgh, Pennsylvania 15237 (US); WRIGHT, Cornell, III., Pittsburgh, Pennsylvania 15206 (US); DARISSE, Ian, Allston, Massachusetts 02134 (US)
(74) Representative: Bittner, Bernhard
(86) International application number: PCT/US2012/040414
(87) International publication number: WO 2012/167043

(56) References cited:
- WO-A2-2010/050771
- US-A1- 2003 135 203
- US-A1- 2004 193 146
- US-A1- 2004 193 146
- US-A1- 2005 093 821
- US-A1- 2009 030 428
- US-A1- 2009 171 151
- US-A1- 2009 326 556
- US-A1- 2010 022 825

## Description

### RELATED APPLICATIONS

This application claims the benefit of U.S. Provisional Application No. 61/492,578, filed June 2, 2011.

### TECHNICAL FIELD

The present inventive concepts generally relate to the field of robotic systems, and more particularly, to articulating robotic systems, robotic system user interfaces, human interface devices for controlling robotic systems and methods of controlling robotic systems.

### BACKGROUND

As less invasive medical techniques and procedures become more widespread, medical professionals, such as surgeons, may employ snake-like robotic systems having highly articulated multi-link probes to access parts of the human anatomy that were otherwise difficult to reach. With the use of such robotic systems, medical professionals may be able to replace open-cavity surgical procedures with less invasive procedures.

US 2009/0326556 discloses a surgical robotic system comprising an articulated probe, at least one surgical tool and a human interface device.

Robotic systems of the type may have multiple device channels for guiding a variety of surgical and/or interventional tools during surgical procedures; however, these tools generally comprise hand operated levers and handles, which are separate from the control device of the robotic system. Thus, medical professionals must physically move between the hand operated levers and handles of the surgical tools and the control device of the robotic system during medical procedures.

### SUMMARY

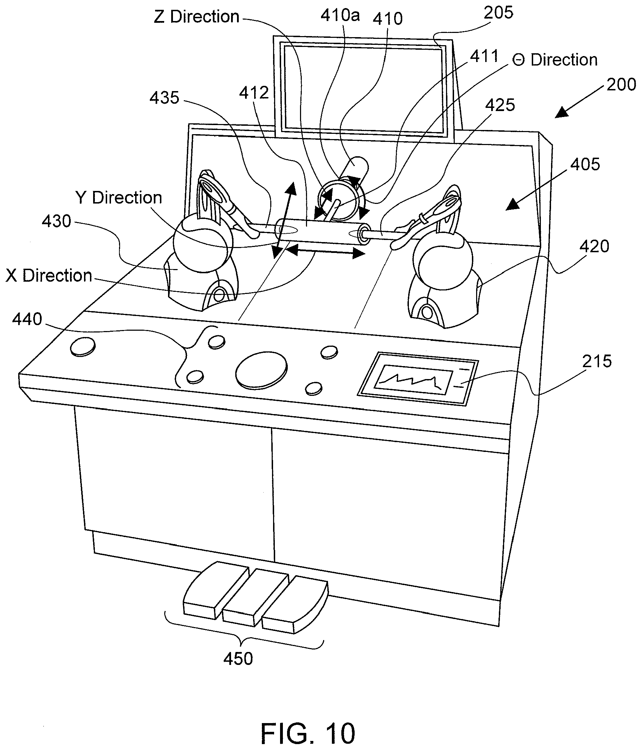

The invention is defined by independent claim 1. An embodiment of the invention is shown in figure 10.

Embodiments of the present disclosure may be directed to articulating robotic systems, robotic system user interfaces, human interface devices for controlling robotic systems and methods of controlling robotic systems.

In one aspect, a system, comprises: an articulating probe; a surgical tool; a controller constructed and arranged to manipulate at least one of the articulating probe and the surgical tool; and a human interface device (HID) configured to generate a first control signal and a second control signal, wherein the articulating probe is manipulated in response to the first control signal and the surgical tool is manipulated in response to the second control signal.

In some embodiments, the articulating probe includes an inner core having a plurality of inner links and an outer sleeve having a plurality of outer links.

In some embodiments, the inner core is at least partially positioned within the outer sleeve.

In some embodiments, the electromechanical feeder system is constructed and arranged to alternately advance or retract the inner core and the outer sleeve.

In some embodiments, the controller is constructed and arranged to advance and retract the articulating probe in response to the first control signal.

In some embodiments, the controller is constructed and arranged to steer the articulating probe in response to the first control signal.

In some embodiments, the articulating probe includes a light source at a distal end of the articulating probe.

In some embodiments, the light source is activated in response to a third control signal generated by the HID.

In some embodiments, the third control signal is an electrical signal.

In some embodiments, the third control signal is transmitted from the HID to the electromechanical feeder system via a conductive wire.

In some embodiments, the articulating probe includes an image capture device at a distal end of the articulating probe.

In some embodiments, the image capture device is activated in response to a third control signal generated by the HID.

In some embodiments, the third control signal is transmitted from the HID to the electromechanical feeder system via a conductive wire.

In some embodiments, the image capture device transmits an image signal to the HID.

In some embodiments, the controller is constructed and arranged to advance or retract the surgical tool in response to the second control signal.

In some embodiments, the controller is constructed and arranged to steer the articulating probe in response to the first control signal.

In some embodiments, the surgical tool includes a tool shaft having an articulation region.

In some embodiments, the articulation region of the tool shaft includes at least two segment links.

In some embodiments, the surgical tool includes a functional element coupled to a distal end of the tool shaft.

In some embodiments, the controller is constructed and arranged to advance or retract the distal end of the tool shaft with respect to a distal end of the articulating probe.

In some embodiments, the functional element includes one selected from the group consisting of: a grasper, a claw, a cutter, a knife, an ablator, a cauterizer, a drug delivery apparatus, a radiation source, an EKG electrode, a pressure sensor, a blood sensor, a camera, a light source, a snare, a basket, a balloon, a clamp, a magnet, a heating element and a cryogenic element.

In some embodiments, the surgical tool includes an actuator, the actuator coupled to the functional element via mechanical linkage.

In some embodiments, the functional element is activated in response to a third control signal generated by the HID.

In some embodiments, the actuator applies a force to the mechanical linkage in response to the third control signal.

In some embodiments, the functional element is activated in response to a third control signal generated by the HID.

In some embodiments, the third control signal is an electrical signal.

In some embodiments, the third control signal is transmitted from the HID to at least one of the controller or the surgical tool via a conductive wire.

In some embodiments, the third control signal is a mechanical signal.

In some embodiments, the third control signal is transferred from the HID to at least one of the controller or the surgical tool via an actuating cable.

In some embodiments, the actuating cable is coupled to the functional element.

In some embodiments, the actuating cable is coupled to mechanical linkage of the functional element.

In some embodiments, the actuating cable is coupled to a lever of the HID.

In some embodiments, a resistance applied to the functional element in response to the third control signal is transferred from the functional element to the lever.

In some embodiments, the third control signal includes a mechanical force that is transferred from the HID to the functional element via an actuating cable.

In some embodiments, the controller advances or retracts the articulating probe in response to the first control signal, and wherein the controller advances or retracts the surgical tool in response to the second control signal.

In some embodiments, the controller steers the articulating probe in response to the first control signal, and wherein the controller steers the surgical tool in response to the second control signal.

In some embodiments, the controller simultaneously manipulates the articulating probe and the surgical tool.

In some embodiments, the controller sequentially manipulates the articulating probe and the surgical tool.

In some embodiments, the controller includes an electromechanical feeder system.

In some embodiments, the electromechanical feeder system advances or retracts the articulating probe in response to the first control signal, and wherein the electromechanical feeder system advances or retracts the surgical tool in response to the second control signal.

In some embodiments, the electromechanical feeder system steers the articulating probe in response to the first control signal, and wherein the electromechanical feeder system steers the surgical tool in response to the second control signal.

In some embodiments, the electromechanical feeder system simultaneously manipulates the articulating probe and the surgical tool.

In some embodiments, the electromechanical feeder system sequentially manipulates the articulating probe and the surgical tool.

In some embodiments, the controller includes a first feeding device constructed and arranged to control the manipulation of the articulating probe.

In some embodiments, the controller includes a first feeding device constructed and arranged to control advancement and retraction of the articulating probe.

In some embodiments, the first feeding device includes at least one actuator constructed and arranged to advance or retract an inner core and an outer sleeve of the articulating probe.

In some embodiments, the first feeding device includes at least one actuator constructed and arranged to configure an inner core of the articulating probe in one of a limp or rigid mode.

In some embodiments, the at least one actuator is constructed and arranged to configure an outer sleeve of the articulating probe in one of the limp or rigid mode,

In some embodiments, the first feeding device includes at least one actuator constructed and arranged to steer at least one of an inner core and an outer sleeve of the articulating probe.

In some embodiments, the first feeding device includes a plurality of actuators.

In some embodiments, the at least one actuator includes an actuator selected from the group consisting of: a linear actuator, a rotary actuator and a solenoid.

In some embodiments, the controller includes a second feeding device constructed and arranged to control the manipulation of the surgical tool.

In some embodiments, the controller includes a second feeding device constructed and arranged to control the advancement and retraction of the surgical tool.

In some embodiments, the second feeding device includes at least one actuator constructed and arranged to advance or retract a shaft of the surgical tool.

In some embodiments, the second feeding device includes at least one actuator constructed and arranged to articulate an articulation region of the surgical tool.

In some embodiments, the second feeding device includes at least one actuator constructed and arranged to actuate a functional element of the surgical tool.

In some embodiments, the second feeding device includes a plurality of actuators.

In some embodiments, the at least one actuator includes an actuator selected from the group consisting of: a linear actuator, a rotary actuator and a solenoid.

In some embodiments, the HID includes at least 4-axes of movement.

In some embodiments, the HID includes a hand-operated control device coupled to a control stick and wherein the control stick is movably coupled to a base.

In some embodiments, the hand-operated control device is coupled to a proximal end of the control stick and wherein a distal end of the control stick is movably coupled to the base.

In some embodiments, the hand-operated control device is moveable with respect to the base in a first-axis of movement.

In some embodiments, the hand-operated control device is moveable with respect to the base in a second axis of movement.

In some embodiments, the first-axis of movement is different from the second axis of movement.

In some embodiments, a joystick of the hand-operated control device is moveable with respect to the hand-operated control device in a third axis of movement.

In some embodiments, the joystick of the hand-operated control device is moveable with respect to the hand-operated control device in a fourth axis of movement.

In some embodiments, the third axis of movement is different from the second axis of movement.

In some embodiments, the joystick of the hand-operated control device is moveable with respect to the hand-operated control device in a fifth axis of movement.

In some embodiments, the fifth axis of movement is different from the third axis of movement and the fourth axis of movement.

In some embodiments, the hand-operated control device is moveable with respect to the base in a sixth axis of movement.

In some embodiments, the sixth axis of movement is different from the first axis of movement and the second axis of movement.

In some embodiments, the HID includes a hand-operated control device.

In some embodiments, the HID further includes a control stick and a base.

In some embodiments, the base of the HID is coupled to the system console.

In some embodiments, the base of the HID is directly coupled to the system console.

In some embodiments, the base of the HID is fixedly attached to the system console.

In some embodiments, the base of the HID is coupled to a table.

In some embodiments, the base of the HID is directly coupled to the table.

In some embodiments, the base of the HID is fixedly attached to the table.

In some embodiments, the table is an operating table.

In some embodiments, the electromechanical feeder system is coupled to the table via a support.

In some embodiments, the hand-operated control device is coupled to a proximal end of the control stick, and wherein the base is slidably coupled to a distal end of the control stick.

In some embodiments, the hand-operated control device is movable with respect to the base.

In some embodiments, the hand-operated control device is moveable with respect to the base in at least two-directions of movement.

In some embodiments, the hand-operated control device is moveable with respect to the base in at least three-directions of movement.

In some embodiments, the hand-operated control device is moveable with respect to the base in four-directions of movement.

In some embodiments, the hand-operated control device is movable with respect to the base in a direction corresponding to a longitudinal axis of the control stick.

In some embodiments, the HID is configured to generate the first control signal in response to a movement of the hand-operated control device in the direction corresponding to the longitudinal axis of the control stick.

In some embodiments, the controller is constructed and arranged to manipulate the articulating probe in response to the first control signal.

In some embodiments, the controller is constructed and arranged to adjust a position of extension of the articulating probe in response to a displacement of the of the hand-operated control device in the direction corresponding to the longitudinal axis of the control stick.

In some embodiments, the controller is constructed and arranged to adjust a velocity of movement of the articulating probe in response to a displacement of the of the hand-operated control device in the direction corresponding to the longitudinal axis of the control stick.

In some embodiments, the hand-operated control device is movable with respect to the base in a direction transverse to a longitudinal axis of the control stick.

In some embodiments, the HID is configured to generate the first control signal in response to a movement of the hand-operated control device in the direction transverse to the longitudinal axis of the control stick.

In some embodiments, the controller is constructed and arranged to steer the articulating probe in response to the first control signal.

In some embodiments, the hand-operated control device includes at least one joystick.

In some embodiments, a first joystick of the at least one joystick is configured to generate the second control signal.

In some embodiments, the controller is constructed and arranged to advance or retract the surgical tool in response to the second control signal.

In some embodiments, the controller is constructed and arranged to adjust a position of extension of the surgical tool in response to a displacement of the first joystick in the direction corresponding to a longitudinal axis of the joystick.

In some embodiments, the controller is constructed and arranged to adjust a velocity of movement of the surgical tool in response to a displacement of the first joystick in the direction corresponding to a longitudinal axis of the control stick.

In some embodiments, a second joystick of the at least one joystick is configured to generate a fourth control signal.

In some embodiments, the controller is constructed and arranged to advance or retract a second surgical tool in response to the fourth control signal.

In some embodiments, the at least one joystick is movable with respect to the hand-operated control device in a direction corresponding to a longitudinal axis of the joystick.

In some embodiments, the hand-operated control device is configured to generate the second control signal in response to a movement of the joystick in the direction corresponding to the longitudinal axis of the joystick.

In some embodiments, the controller is constructed and arranged to advance or retract the surgical tool in response to the second control signal.

In some embodiments, the at least one joystick is movable with respect to the hand-operated control device in a direction transverse to a longitudinal axis of the joystick.

In some embodiments, the hand-operated control device is configured to generate the second control signal in response to a movement of the joystick in the direction corresponding to the longitudinal axis of the joystick.

In some embodiments, the controller is constructed and arranged to articulate an articulation region of the surgical tool in response to the second control signal.

In some embodiments, the hand-operated control device includes a lever.

In some embodiments, a first end of an actuating cable is coupled to the lever and a second end of the actuating cable is coupled to a functional element of the surgical tool.

In some embodiments, the second end of the actuating cable is coupled to mechanical linkage of the functional element.

In some embodiments, the lever is constructed and arranged to apply a tension to the actuating cable.

In some embodiments, the functional element is actuated in response to the tension applied to the actuating cable.

In some embodiments, the hand-operated control device includes an actuator.

In some embodiments, the actuator includes an actuator selected from the group consisting of: a linear actuator and a solenoid.

In some embodiments, the actuator is constructed and arranged to apply a tension to the actuating cable.

In some embodiments, the actuator is constructed and arranged to apply a tension to the actuating cable in response to an actuating control signal.

In some embodiments, the hand-operated control device includes a force sensing button that generates the actuating control signal.

In some embodiments, a magnitude of tension applied to the actuating cable is proportional to a force applied to the force sensing button.

In some embodiments, the hand-operated control device includes a button.

In some embodiments, the button includes a force sensing resistor.

In some embodiments, the hand-operated control device includes a vibration transducer.

In some embodiments, the vibration transducer is activated in response to one of the articulating probe and the surgical tool abutting a cavity wall of a patient.

In some embodiments, the hand-operated control device includes a first set of push buttons.

In some embodiments, a first button of the first set of push buttons is configured to map directional movements of a first joystick of the hand-operated control device to electromechanical movements of the surgical tool.

In some embodiments, a second button of the first set of push buttons is configured to actuate a functional element of the surgical tool.

In some embodiments, the hand-operated control device includes a second set of push buttons.

In some embodiments, a first button of the second set of push buttons is configured to map directional movements of a second joystick of the hand-operated control device to electromechanical movements of a second surgical tool.

In some embodiments, a second button of the second set of push buttons is configured to actuate a functional element of the second surgical tool.

In some embodiments, the hand-operated control device includes a mode selection switch.

In some embodiments, the mode selection switch is configured to map directional movements of a first joystick of the hand-operated control device to electromechanical movements of the surgical tool in a first mode.

In some embodiments, the mode selection switch is configured to map directional movements of a first joystick of the hand-operated control device to electromechanical movements of the articulating probe in a second mode.

In some embodiments, the hand-operated control device includes a display device.

In some embodiments, the display device includes one selected from the group consisting of a liquid crystal display, an organic light-emitting diode display, a light-emitting diode display, an electronic ink display and an electrophoretic display.

In some embodiments, the display device is configured to display an image captured by a camera of the articulating probe.

In some embodiments, the display device is configured to display a live video stream transmitted by a camera of the articulating probe.

In some embodiments, the display device is configured to prompt a user of the system.

In some embodiments, the display device is configured to prompt a user of the system with one or more steps of a medical procedure.

In some embodiments, the display device is configured to inform a user of the system with one or more patient vitals.

In some embodiments, the system further comprises a second surgical tool.

In some embodiments, the second surgical tool comprises: a functional element at a distal end of a tool shaft; a hand-operated lever at a proximal end of the tool shaft, wherein the hand-operated lever is constructed and arranged to actuate the functional element.

In some embodiments, the second surgical tool includes a joystick.

In some embodiments, the joystick is integral with the hand operated lever.

In some embodiments, the joystick is removably coupled to the second surgical tool.

In some embodiments, the joystick is affixed to the second surgical tool with an adhesive.

In some embodiments, the joystick is secured to the second surgical tool.

In some embodiments, the joystick is configured to generate the first control signal.

In some embodiments, the joystick is configured to generate the first control signal in response to a movement of the joystick.

In some embodiments, the HID includes a first multi-axis input device and a second multi-axis input device.

In some embodiments, the HID is coupled to a console.

In some embodiments, the console includes at least one foot pedal.

In some embodiments, the at least one foot pedal is configured to generate a third control signal.

In some embodiments, a functional element of the surgical is activated in response to the third control signal.

In some embodiments, the HID is configured to generate the first control signal when a first user interface of the first multi-axis input device and a second user interface of the second multi-axis input device are coupled, and wherein the HID is configured to generate the second control signal when the first user interface of the first multi-axis input device and the second user interface of the second multi-axis input device are decoupled.

In some embodiments, the first user interface includes a stylus.

In some embodiments, the second user interface includes a stylus.

In some embodiments, the electromechanical feeder system advances or retracts the articulating probe in response to the first control signal, and wherein the electromechanical feeder system advances or retracts the surgical tool in response to the second control signal.

In some embodiments, HID includes a coupling member constructed and arranged to removably couple the first user interface with the second user interface.

In some embodiments, the HID is configured to generate the first control signal when a first user interface of the first multi-axis input device and a second user interface of the second multi-axis input device are synchronously moved.

In some embodiments, the HID is configured to generate the second control signal when the first user interface of the first multi-axis input device and the second user interface of the second multi-axis input device are independently moved.

In some embodiments, a first user interface of the first multi-axis input device is moveable in at least three degrees of freedom, and wherein a second user interface of the second multi-axis input device is moveable in at least three degrees of freedom.

### BRIEF DESCRIPTION OF THE DRAWINGS

The foregoing and other objects, features and advantages of embodiments of the present inventive concepts will be apparent from the more particular description of preferred embodiments, as illustrated in the accompanying drawings in which like reference characters refer to the same elements throughout the different views. The drawings are not necessarily to scale, emphasis instead being placed upon illustrating the principles of the preferred embodiments.
**FIG. 1** is a perspective view of an articulating robotic system, in accordance with embodiments of the present disclosure ;
**FIG. 2** is a perspective view of an articulating robotic system, in accordance with embodiments of the present disclosure;
**FIG. 3** is a perspective view of an articulating robotic system performing a medical procedure, in accordance with embodiments of the present disclosure;
**FIG. 4** is a diagrammatic view of the articulating robotic systems shown in FIGs. 1 and 2, in accordance with embodiments of the present disclosure;
**FIGs. 5A** **and** **5B** are perspective views of the hand operated control device shown in FIGs. 1, 2 and 4, in accordance with embodiments of the present disclosure;
**FIGs. 6A-6C** are perspective views of hand operated control devices, in accordance with embodiments of the present disclosure;
**FIG. 7** is a perspective view of the human interface device shown in FIGs. 1, 2, 4, 5A and 5B, in accordance with embodiments of the present disclosure;
**FIGs. 8A and 8B** are sectional views of an electrically activated surgical tool, in accordance with embodiments of the present disclosure;
**FIGs. 9A and 9B** are sectional views of a mechanically activated surgical tool, in accordance with embodiments of the present disclosure;
**FIG. 10** is a perspective view of a console system, in accordance with embodiments of the present invention;
**FIG. 11** is a perspective view of a surgical tool, in accordance with embodiments of the present disclosure.

### DETAILED DESCRIPTION OF EMBODIMENTS

The terminology used herein is for the purpose of describing particular embodiments and is not intended to be limiting of the inventive concepts. As used herein, the singular forms "a," "an" and "the" are intended to include the plural forms as well, unless the context clearly indicates otherwise. It will be further understood that the terms "comprises," "comprising," "includes" and/or "including," when used herein, specify the presence of stated features, integers, steps, operations, elements, and/or components, but do not preclude the presence or addition of one or more other features, integers, steps, operations, elements, components, and/or groups thereof.

It will be understood that, although the terms first, second, third etc. may be used herein to describe various limitations, elements, components, regions, layers and/or sections, these limitations, elements, components, regions, layers and/or sections should not be limited by these terms. These terms are only used to distinguish one limitation, element, component, region, layer or section from another limitation, element, component, region, layer or section. Thus, a first limitation, element, component, region, layer or section discussed below could be termed a second limitation, element, component, region, layer or section without departing from the teachings of the present application.

It will be further understood that when an element is referred to as being "on" or "connected" or "coupled" to another element, it can be directly on or above, or connected or coupled to, the other element or intervening elements can be present. In contrast, when an element is referred to as being "directly on" or "directly connected" or "directly coupled" to another element, there are no intervening elements present. Other words used to describe the relationship between elements should be interpreted in a like fashion (e.g., "between" versus "directly between," "adjacent" versus "directly adjacent," etc.). When an element is referred to herein as being "over" another element, it can be over or under the other element, and either directly coupled to the other element, or intervening elements may be present, or the elements may be spaced apart by a void or gap.

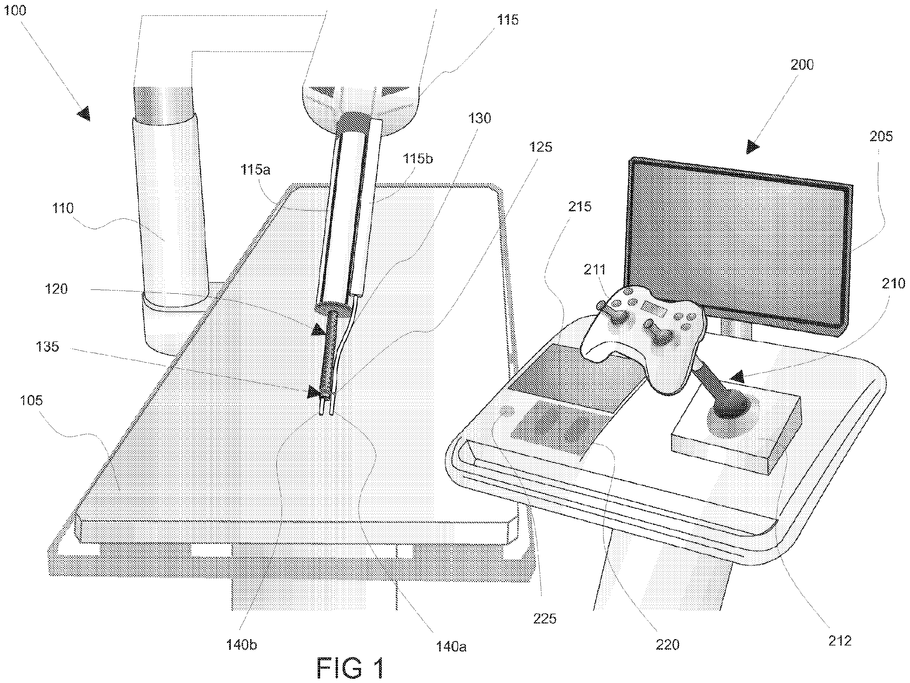

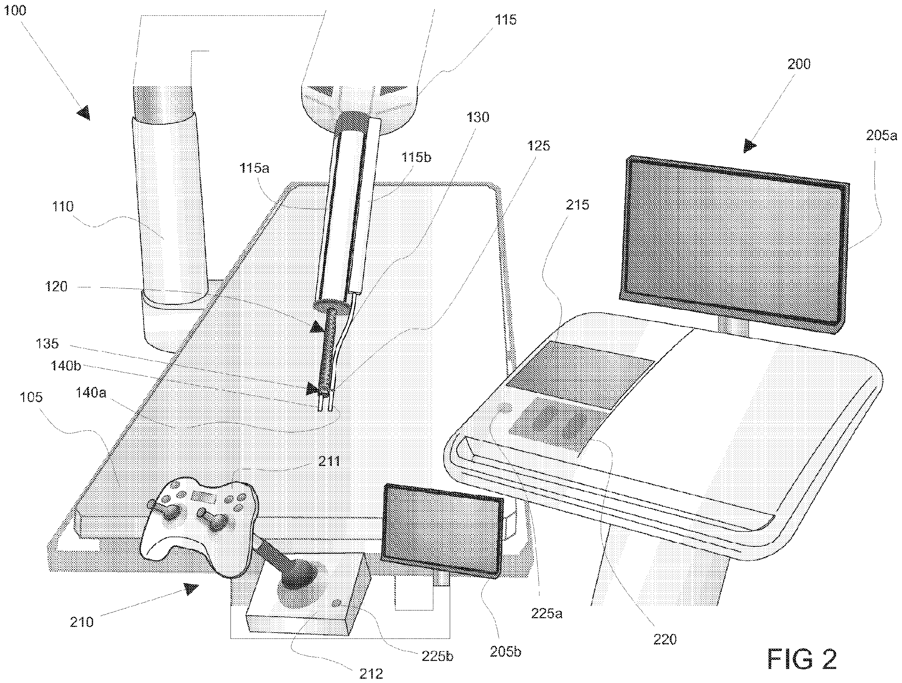

**FIG. 1** is a perspective view of an articulating robotic system, and **FIG. 2** is a perspective view of another articulating robotic system. A system 100, such as an articulating robotic system and/or a system for performing a medical procedure (e.g., transoral robotic surgery procedure) may include an articulating probe 120, one or more surgical tools 140, 140a-b, a controller 115 and a human interface device (HID) 210.

The system 100 may include one or more features of the surgical positioning and support system described in U.S. Provisional Patent Application Serial No. 61/368,257, filed July 28, 2010, and the systems and tools described in U.S. Provisional Application No. 61/472,344, filed April 6, 2011, the contents of each application being incorporated by reference herein in their entirety.

The articulating probe 120 may include an inner sleeve (not shown) and an outer sleeve, which can advance or retract with respect to one another during manipulation of the articulating probe 120. For example, the inner and outer sleeves of the articulating probe 120, which may include a plurality of inner links and a plurality of outer links, can be configured in one of a limp mode and a rigid mode so as to facilitate the manipulation of the articulating probe 120.

Exemplary probes are further described in U.S. Patent Application Publication No. 2009/0171151, published on July 2, 2009, by Choset, et al., and U.S. Patent Application Publication No. 2008/0039690, published February 14, 2008, by Zubiate, et al., the contents of each being herein incorporated by reference in their entirety.

The articulating probe 120 may include at least one working channel having an opening at a distal end 135 of the articulating probe 120. The working channel may extend throughout the articulating probe 120, for example, from a proximal end to a distal end 135 of the articulating probe 120. In this manner, one or more surgical tools 140, 140a-b may be slidably positioned within the working channel of the articulating probe 120. For example, in the embodiments shown in FIGs. 1 and 2, a second surgical tool 140b is shown positioned within a working channel of the articulating probe 120.

The articulating probe 120 may include at least one side port or guide hole 125. For example, in the embodiments shown at FIGs. 1 and 2, the articulating probe 120 includes a first side port 125 formed in flanges of an outer link of the articulating probe 120. The articulating probe 120 may further include at least one feed tube 130 coupled to the side port or guide hole 125 of the articulating probe 120. In this manner, one or more surgical tools 140, 140a-b may be slidably positioned within the side port 125 of the articulating probe 120. For example, in the embodiments shown in FIGs. 1 and 2, a first surgical tool 140a is shown positioned within the side port 125 of the articulating probe 120.

The system 100 may include one or more surgical tools 140, 140a-b having an articulation region. The system 100 may be configured to allow an operator to independently control the articulating probe 120 and the surgical tools 140, 140a-b. For example, the articulating probe 120 and the surgical tools 140, 140a-b may be controlled independently via the HID 210. The system 100 may be configured with any number of surgical tools 140, 140a-b, which can be slidably positioned within the working channel of the articulating probe 120 and/or the side port or guide hole 125 of the articulating probe 120.

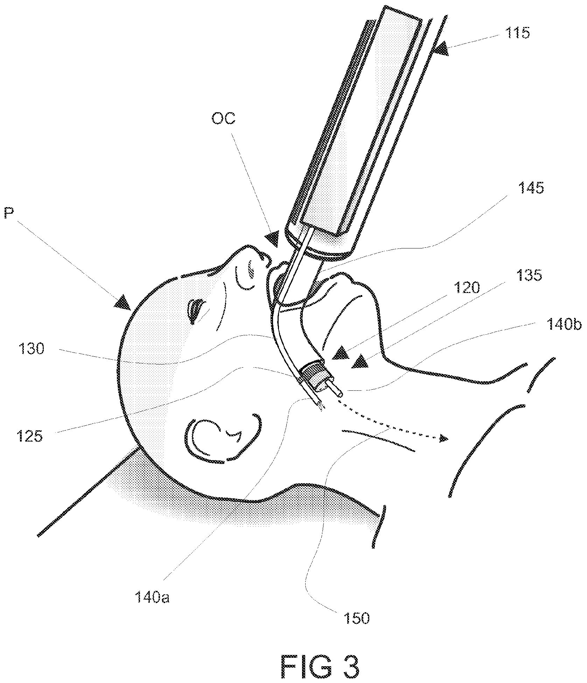

The articulating probe 120 may be constructed and arranged to guide one or more surgical tools 140, 140a-b and/or tool sheaths within a patient body (see for example, FIG. 3), and the controller 115 may be constructed and arranged to manipulate at least one of the articulating probe 120 and the surgical tool 140, 140a-b. For example, the controller 115 may be constructed and arranged to advance or retract the articulating probe 120, advance or retract the surgical tool 140, 140a-b, steer the articulating probe 120, steer the surgical tool 140, 140a-b and/or activate a functional element of the surgical tool 140, 140a-b.

An operator, such as a medical professional, may control the articulating probe 120 and/or the surgical tools 140, 140a-b via the human interface device (HID) 210, which may be configured to generate one or more control signals. For example, the HID 210 may be configured to generate a first control signal and a second control signal. The articulating probe 120 may be manipulated in response to the first control signal, and the surgical tool 140, 140a-b may be manipulated in response to the second control signal.

The controller 115 may be constructed and arranged to manipulate or otherwise control the functions and/or movements of the articulating probe 120 and the surgical tools 140, 140a-b. The controller may advance or retract the articulating probe 120 in response to the first control signal and may advance or retract the surgical tool(s) 140, 140a-b in response to the second control signal. The controller 115 may be constructed and arranged to steer the articulating probe 120 in response to the first control signal and may steer the surgical tool(s) 140, 140a-b in response to the second control signal. The controller 115 may simultaneously manipulate the articulating probe 120 and the surgical tool(s) 140, 140a-b. The controller may sequentially manipulate the articulating probe 120 and the surgical tool(s) 140, 140a-b.

The controller 115 may include an electromechanical feeder system, which may be constructed and arranged to manipulate or otherwise control the functions and/or movements of the articulating probe 120 and the surgical tools 140, 140a-b. The electromechanical feeder system may include one or more features of the feeder system described in U.S. Provisional Application No. 61/412,733, filed November 11, 2010, the contents of which is incorporated by reference herein in its entirety.

The controller 115 may be coupled to a table 105 (e.g., operating table) via a support 110; however, in other embodiments the controller 115 may be coupled to a floor standing support. The controller 115 may be electrically coupled or wirelessly connected to the HID 210.

The system 100 may further include a console 200. In various embodiments, the base 212 of the HID 210 may be coupled, fixedly attached or directly coupled to the console 200 or the table 105. For example, in the embodiment shown in FIG. 1, the base 212 of HID 210 is coupled to the console 200, and in the embodiment shown in FIG. 2, the base 212 of the HID 210 is coupled to the table 105.

The console 200 may include a monitor 205, 205a-b, which may be configured to display images and/or sensor readings from devices (e.g., functional elements, cameras, probes, sensors) coupled to or provided with the articulating probe 120 and/or the surgical tools 140, 140a-b. A monitor 205b may further be coupled to the table 105.

The console 200 may further include a graphical user interface (GUI) 215, which may display numerous system, patient, procedure and other information including but not limited to: system state; alarm or warning state; status of robotic control (e.g. advancing, retracting, steering and paused), status of tool (e.g. graspers closed, energy being delivered, steering) medical/surgical procedure information and/or patient vitals. The console 200 may further include an input device 220, such as a keyboard, touch screen, touch pad and/or pointing device. The console 200 and/or base 212 of the HID 210 may include an emergency stop button 225a and/or 225b that may initiate an emergency procedure and/or disable the system 200. For example, upon activation of the emergency stop button(s) 225a and/or 225b, the system 200 may initiate a retraction procedure that removes the articulating probe 120 from within a patient body. Alternatively, emergency stop button(s) 225a and/or 225b may be configured to transition probe 120 to a limp state or a rigid state.

**FIG. 3** is a perspective view of an articulating robotic system performing a medical procedure. The articulating probe 120 of the system 100 is shown advancing along a path 150 within an oral cavity OC of a patient P. In this exemplarily embodiment, the system 100 includes an optional introduction device 145, which is coupled to a distal end of the controller 115. The introduction device 145 can be configured to improve and/or expedite the introduction or removal of the articulating probe 120 from within the patient's P body.

The system 100 and/or introduction device 145 may include one or more features of the system and/or introduction device described in U.S. Provisional Patent Application Serial No. 61/412,733, filed November 11, 2010, the content of which is incorporated by reference herein in its entirety.

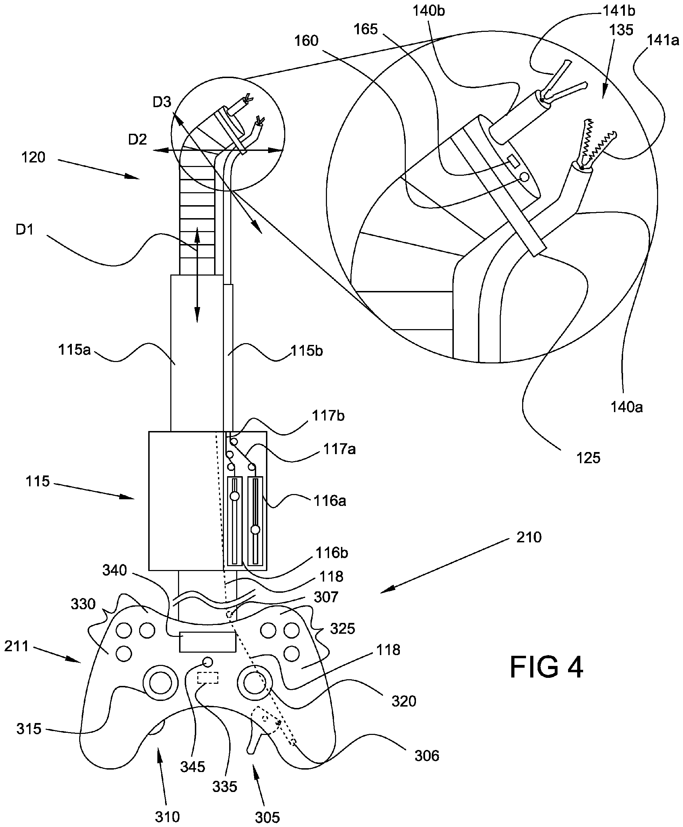

**FIG. 4** is a diagrammatic view of the articulating robotic systems shown in FIGs. 1 and 2, and **FIGs. 5A** **and** **5B** are perspective views of the hand operated control device shown in FIGs. 1, 2 and 4. The controller 115 may include a first feeding device 115a constructed and arranged to control the manipulation of the articulating probe 120, and may further include a second feeding device 115b constructed and arranged to control the manipulation of the surgical tool(s) 140, 140a-b.

The first feeding 115a device may include one or more actuators constructed and arranged to advance or retract an inner core and an outer sleeve of the articulating probe 120. The one or more actuators of the first feeding device 115a may be constructed and arranged to configure the inner core of the articulating probe 120 in one of a limp or rigid mode. The one or more actuators of the first feeding device 115a may be constructed and arranged to configure the outer sleeve of the articulating probe 120 in one of the limp or rigid mode. The one or more actuators of the first feeding device 115a may be constructed and arranged to steer at least one of the inner core and the outer sleeve of the articulating probe 120.

The first feeding device 115a may include one or more actuators constructed and arranged to advance or retract a shaft of the surgical tool 140b. The one or more actuators may be constructed and arranged to articulate an articulation region of the surgical tool. 140b. The one or more actuators may be constructed and arranged to actuate a functional element of the surgical tool 140b.

The first feeding device 115a may include a plurality of actuators. The first feeding device 115a may include an actuator selected from the group consisting of: a linear actuator, a rotary actuator and a solenoid.

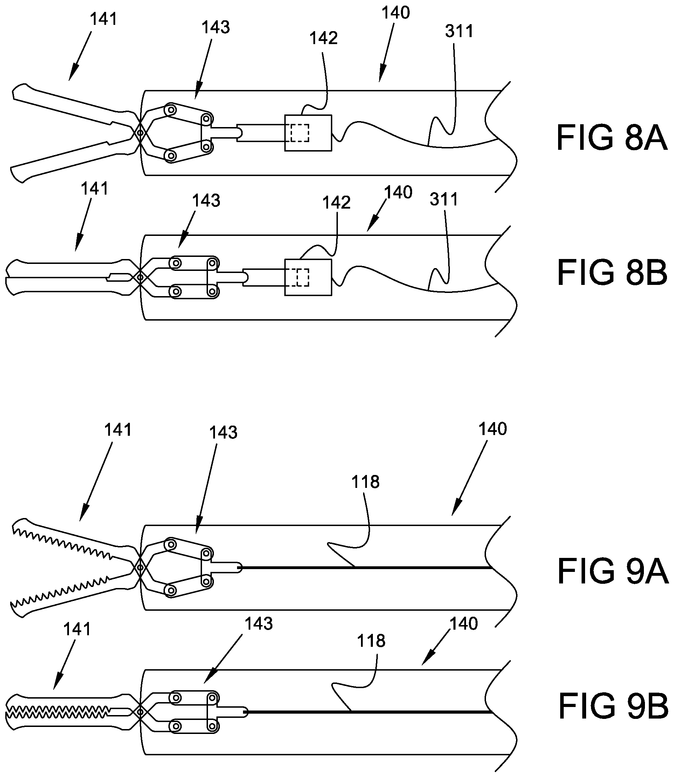

The second feeding device 115b may be constructed and arranged to control the advancement and retraction of the surgical tool. The second feeding device 115b may include one or more actuators 116, 116a-b constructed and arranged to advance or retract a shaft of the surgical tool 140a. The one or more actuators 116, 116a-b may be constructed and arranged to articulate an articulation region of the surgical tool 140a. For example, the first actuator 116a may be constructed and arranged to apply a tension to a first steering cable 117a. The one or more actuators 116, 116a-b may be constructed and arranged to actuate a functional element of the surgical tool 140a. For example, the second actuator 116b may be constructed and arranged to apply a tension to a first actuating cable 117b (such as the actuating cable 118 shown in FIGs. 9A and 9B).

The second feeding device 115b may include a plurality of actuators. The second feeding device 115b may include an actuator selected from the group consisting of: a linear actuator, a rotary actuator and a solenoid.

The articulating probe 120 may include one or more light sources 165 provided at the distal end 135 of the articulating probe 120. The light sources 165 may include electron stimulated light sources such as electron stimulated luminescence light sources, incandescent light sources such as incandescent light bulbs, electroluminescent light sources such as light-emitting diodes, laser light sources such as laser diode light sources, and gas discharge light sources such as fluorescent lamps. The light sources 165 may further include optical fibers, which can be configured to transmit light to and from the distal end 135 of the articulating probe 120.

The light source 165 may be activated in response to a third control signal generated by the HID 210. The third control signal may be an electrical signal, which may be transmitted from the HID 210 to the controller via a conductive wire. Light source 165 may be further controlled by additional control signals generated by HID 210. These additional control signals may be used to adjust one or more outputs of light source 165 such as the intensity of light produced, color or type of light produced, or the direction of light delivered.

The articulating probe 120 may include an image capture device 160, such as an optical camera, video camera, IR camera or other type of camera system. The image capture device 160 may be provided at the distal end 135 or working surface of the articulating probe 120. The image capture device 160 may be activated in response to a fourth control signal generated by the HID 210, which may be transmitted from the HID 210 to the controller 115 via a conductive wire. In response to the fourth control signal, the image capture device 160 may transmit an image signal, such as a picture or video to the HID 210 and/or the console 200.

The surgical tools 140, 140a-b may include functional elements 141, 141a-b. The functional elements 141, 141a-b may be constructed and arranged to articulate with respect to the distal end 135 or working surface of the articulating probe 120. The functional elements 141, 141a-b may also be constructed and arranged to articulate with respect to an axis of extension of the tool shaft.

The functional elements 141, 141a-b may include one or more selected from the group consisting of: a grasper, a claw, a cutter, a knife, an ablator, a cauterizer, a drug delivery apparatus, a radiation source, an EKG electrode, a pressure sensor, a blood sensor, a camera, a light source, a snare (e.g. for polyp removal), a basket (e.g. for removing tissue), a balloon, a clamp, a magnet, a heating element and a cryogenic element. For example, the functional element 141, 141a-b may include a cutter having first and second blades. The functional element 141, 141a-b may include a heating element, cryogenic element, a pressure sensor, a blood sensor and/or a radiation source. The functional element 141, 141a-b may include one or more EKG electrodes or heart defibrillator electrodes. The functional element 141, 141a-b may include a camera.

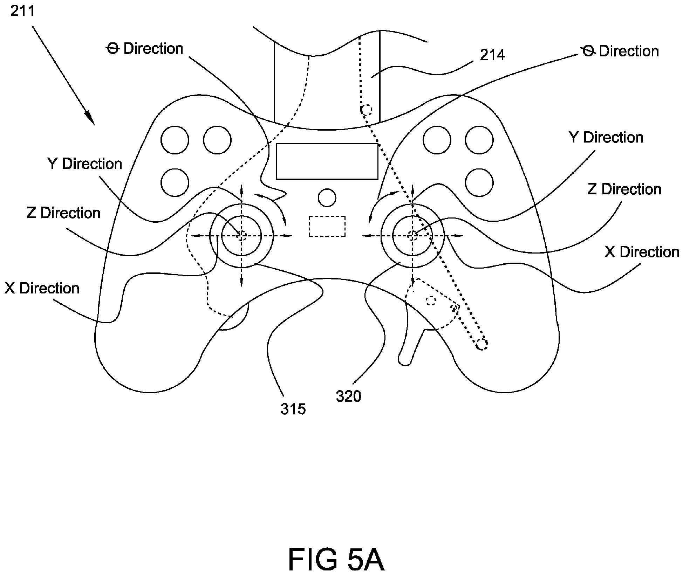

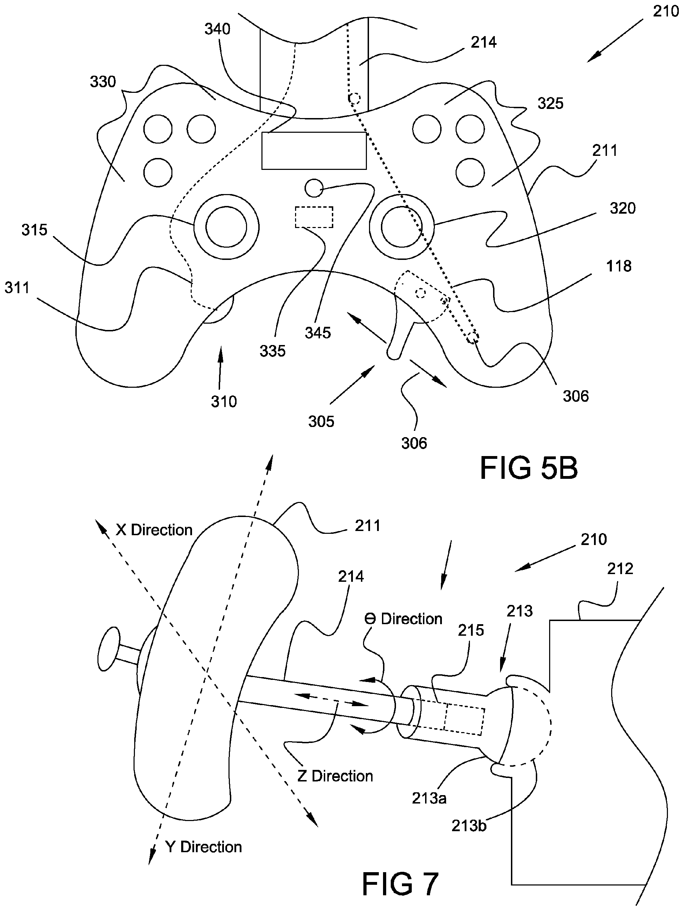

The HID 210 may include a hand-operated control device 211, and the hand-operated control device 211 may include one or more joysticks 315, 320. The joysticks 315, 320 may be movable with respect to the hand-operated control device 211. For example, the joysticks 315, 320 may be moveable with respect to the hand-operated control device 211 in at least one or more of the directions selected from the group consisting of: the X direction, the Y direction, the Z direction and the θ direction (see for example, the X direction, the Y direction and the Z direction illustrated in FIG. 5A).

Movements of the one or more joysticks 315, 320 with respect to the hand-operated control device 211 may generate control signals for manipulating one or more of the surgical tools 140, 140a-b of the system 100. For example, a movement of one of the joysticks 315, 320 with respect to the hand-operated control device 211 in the X direction may manipulate and/or articulate an articulation region of one of the surgical tools 140, 140a-b in a second direction D2 (see for example, the second direction D2 illustrated in FIG 4). A movement of one of the joysticks 315, 320 with respect to the hand-operated control device 211 in the Y direction may manipulate and/or articulate an articulation region of one of the surgical tools 140, 140a-b in a third direction D3 (see for example, the third direction D3 illustrated in FIG 4). A movement of one of the joysticks 315, 320 with respect to the hand-operated control device 211 in the Z direction may manipulate and/or advance or retract one of the surgical tools 140, 140a-b in a first direction D1 (see for example, the first direction D1 illustrated in FIG 4). Alternatively or additionally, one or more input devices, such as hand-operated control device 211 may be constructed and arranged to move, such that movement of the entire input device can be used to generate a control signal (e.g. via integral sensors such as accelerometers).

A first joystick of the one or more joysticks 315, 320 may be configured to generate the second control signal. As described above, the controller 115 may be constructed and arranged to manipulate (e.g., advance or retract, steer, actuate) one of the surgical tools 140, 140a-b in response to the second control signal. For example, the controller 115 may be constructed and arranged to adjust a position of extension of one of the surgical tools 140, 140a-b in response to a displacement of the of the first joystick in the direction corresponding to a longitudinal axis of the joystick. The controller 115 may be constructed and arranged to adjust a velocity of movement of one of the surgical tools 140, 140a-b in response to a displacement of the first joystick in the direction corresponding to a longitudinal axis of the control stick.

A second joystick of the one or more joysticks 315, 320 may be configured to generate a fourth control signal. The controller 115 may be constructed and arranged to manipulate (e.g., advance or retract, steer, actuate) another of the one of the surgical tools 140, 140a-b in response to the fourth control signal.

In some embodiments, displacement of the joystick 315, 320 in any one of the X, Y or Z directions can optionally control displacement, velocity or acceleration movements of a surgical tool 140, 140a-b. For example, displacing one of the joysticks 315, 320 by a first distance in anyone of the X, Y or Z directions may command and/or control a surgical tool 140, 140a-b of the system 100 to be displaced by the first distance (or a multiple thereof) in a corresponding direction (displacement control). In another example, displacing one of the joysticks 315, 320 by a first distance in anyone of the X, Y or Z directions may command and/or control a surgical tool 140, 140a-b of the system 100 to move at a velocity proportional to the first distance of displacement in a corresponding direction (velocity control). In another example, displacing one of the joysticks 315, 320 by a first distance in anyone of the X, Y or Z directions may command and/or control a surgical tool 140, 140a-b of the system 100 to accelerate proportional to the first distance of displacement in a corresponding direction (acceleration control).

Alternatively or additionally, velocity or acceleration of the joystick 315, 320 in anyone of the X, Y or Z directions can optionally control displacement, velocity or acceleration of a surgical tool 140, 140a-b. For example, moving one of the joysticks 315, 320 at a first speed in anyone of the X, Y or Z directions may command and/or control a surgical tool 140, 140a-b of the system 100 to move at a speed proportional to the first speed in a corresponding direction. In another example, moving one of the joysticks 315, 320 at a first speed in anyone of the X, Y or Z directions may command and/or control a surgical tool 140, 140a-b of the system 100 to be displaced by a distance proportional to the first speed in a corresponding direction. Alternatively or additionally, a magnitude of force exerted on a joystick 315, 320 in anyone of the X, Y or Z directions can optionally control displacement, velocity or acceleration of a surgical tool 140, 140a-b. In addition, one or more of the buttons (of the sets of buttons 325, 330) of the hand-operated control device 211 can be configured to toggle between control modes, such as, a displacement control mode, a velocity control mode and an acceleration control mode.

Similarly, other outputs of the HID 210 or input signals of the surgical tool(s) 140, 140a-b and/or the articulating probe 120 can be controlled according to one or more of the above modes of operation. For example, a force exerted by surgical tool 140, 140a-b (e.g. grasper to pull hard on tissue) can be mapped to a force exerted on the hand-operated control device 211 or one of the joysticks 315, 320.

In addition, the HID 210 may be constructed and arranged to provide tactile feedback to a user. For example, the hand-operated control device 211 and/or one or more of the joysticks 315, 320 may be configured to provide mechanical stimulation to assist a user in visualizing movements of the articulating probe 120 and/or surgical tools 140, 140a-b within a patient's body or other type of cavity.

The hand-operated control device 211 my include a button 310, such as a push button or force sensing resistor button, which may be configured to generate one or more control signals for actuating a functional element 141, 141a-b of one of the surgical tools 140, 140a-b.

The hand-operated control device 211 may include a vibration transducer 335 that may be configured to provide haptic or tactile feedback to an operator gripping or holding the hand-operated control device 211. For example, the vibration transducer 335 may provide haptic or tactile feedback to an operator gripping or holding the hand-operated control device 211 so as to notify the operator when an outer surface and/or the distal end 135 of the articulating probe 120 comes into contact with an inner cavity or lumen wall of a patient. Additionally or alternatively, tactile, vibrational, force or other feedback can be use to alert the operator of one or more system states such as an alarm or warning state.

The hand-operated control device may include one or more sets of buttons or switches 325, 330. For example, the hand-operated control device may include a first set of buttons or switches 325 and a second set of buttons or switches 330. In some embodiments, a first button of the first or second sets of buttons 325, 330 may be configured to map directional movements of a first joystick of the one or more joysticks 315, 320 to electromechanical movements of one of the surgical tools 140, 140a-b. A second button of the first or second sets of buttons 325, 330 may be configured to actuate a functional element of one of the surgical tools 140, 140a-b.

The hand-operated control device 211 may include a mode selection switch or button 345, which may be configured to change the control mode of a joystick. In a first control mode, directional movements of a first joystick of the one or more joysticks 315, 320 of the hand-operated control device 211 map to electromechanical movements of one of the surgical tools 140, 140a-b. In a second control mode, directional movements of the first joystick of the one or more joysticks 315, 320 of the hand-operated control device 211 map to electromechanical movements of the articulating probe 120. An operator may toggle between the first and second modes by actuating the mode selection switch or button 345. Additional control modes may be included such as to control an additional tube, or to control articulating probe 120 and/or surgical tools 140, 140a-b in a different manner.

The hand-operated control device 211 may include a display device 340. The display device 340 may include one selected from the group consisting of a liquid crystal display, a CRT, an organic light-emitting diode display, a light-emitting diode display, an electronic ink display and an electrophoretic display. The display device 340 may be configured to display an image captured by a camera 160 of the articulating probe 120. The display device 340 may be configured to prompt a user of the system 100 with one or more steps of a medical procedure.

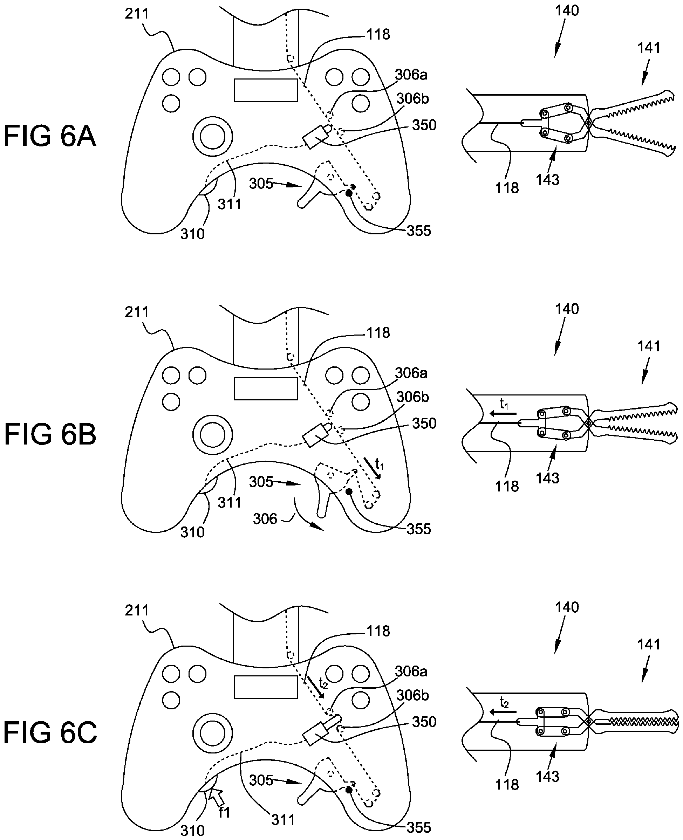

**FIGs. 6A-6C** are perspective views of hand operated control devices. The hand-operated control device 211 may include a lever 305 that is configured to actuate a functional element 141 of a surgical tool 140. For example, a first end of an actuating cable 118 may be coupled to the lever 305 and a second end of the actuating cable 118 may be coupled to a functional element 141 of the surgical tool 140. The actuating cable 118 may extend from the hand-operated control device 211 to the distal end of the surgical tool 140, 140a-b, and may be positioned within elements of the system 100 via one or more posts or capstans 307 (see FIG. 4). In some embodiments, the second end of the actuating cable 118 may be coupled to mechanical linkage 143 of the functional element 141.

The lever 305 may be constructed and arranged to apply a first tension t1 to the actuating cable 118. The functional element 141 may be actuated in response to the first tension t1 applied to the actuating cable 118. For example, an operator holding or gripping the hand-operated control device 211 may apply a force 306, such as a lateral force, to the lever 305 to apply the first tension t1 to the actuating cable 118.

The hand-operated control device may further include an actuator 350, such as a linear actuator or solenoid. The actuator 350 may be constructed and arranged to apply a second tension t2 to the actuating cable 118 in response to an actuating control signal. The hand-operated control device 211 may include a button 310, such as a push button or force sensing button that may generate the actuating control signal. The actuating control signal may be transmitted from the button 310 to the actuator 350 via a wire or cable 311. In embodiments including a force sensing button, a magnitude of the second tension t2 applied to the actuating cable 118 may be proportional to an actuating force fl applied to the button 310.

In the embodiment illustrated at FIGs. 6A-6C, a slidable slug of the actuator 350 advances in an outward direction so as to push a portion of the actuating cable 118 between first and second posts 306a-b. This movement causes the second tension t2 to be applied to actuating cable 118.

The hand-operated control device may further include a stop 355, which may be constructed and arranged to limit movement of the lever 305.

**FIG. 7** is a perspective view of the human interface device shown in FIGs. 1, 2, 4, 5A and 5B. The HID 210 may include a hand-operated control device 211, a control stick 216 and a base 212. The hand-operated control device 211 may be coupled to a proximal end 214 of the control stick 216, and the base 212 may be moveably coupled or slidably coupled to a distal end 215 of the control stick 216.

The hand-operated control device 211 may be movable with respect to the base 212. For example, the hand-operated control device 211 may be moveable with respect to the base 212 in at least one or more of the directions selected from the group consisting of: the X direction, the Y direction, the Z direction and the θ direction.

Movements of the hand-operated control device 211 with respect to the base 212 may generate control signals for manipulating the articulating probe 120 of the system 100. For example, a movement of the hand-operated control device 211 with respect to the base 212 in the X direction may manipulate and/or steer the articulating probe 120 in a second direction D2 (see for example, the second direction D2 illustrated in FIG 4). A movement of the hand-operated control device 211 with respect to the base 212 in the Y direction may manipulate and/or steer the articulating probe 120 in a third direction D3 (see for example, the third direction D3 illustrated in FIG 4). A movement of the hand-operated control device 211 with respect to the base 212 in the Z direction may manipulate and/or advance or retract the articulating probe 120 in a first direction D1 (see for example, the first direction D1 illustrated in FIG 4).

For example, the hand-operated control device 211 may be movable with respect to the base 212 in a direction corresponding to a longitudinal axis of the control stick 216. The direction corresponding to the longitudinal axis of the control stick 216 may correspond to the Z direction. The HID 210 may be configured to generate the first control signal in response to a movement of the hand-operated control device 211 in the direction corresponding to the longitudinal axis of the control stick 216. As described above, the controller 115 of the system 100 may be constructed and arranged to manipulate the articulating probe 120 in response to the first control signal.

In some embodiments, the controller 115 may be constructed and arranged to adjust a position of extension of the articulating probe 120 in response to a displacement of the of the hand-operated control device 211 in the direction corresponding to the longitudinal axis of the control stick 216. The controller 115 may be constructed and arranged to adjust a velocity of movement of the articulating probe 120 in response to a displacement of the of the hand-operated control device 211 in the direction corresponding to the longitudinal axis of the control stick 216.

The hand-operated control device 211 may be movable with respect to the base 212 in a direction transverse to a longitudinal axis of the control stick 216. The direction transverse to a longitudinal axis of the control stick 216 may correspond to the X direction and/or the Y direction. In some embodiments, the hand-operated control device can be coupled to the base at a ball and socket joint 213, including ball 213a and socket 213b. The HID 210 may be configured to generate the first control signal in response to a movement of the hand-operated control device in the direction transverse to the longitudinal axis of the control stick 216. As described above, the controller 115 of the system 100 may be constructed and arranged to manipulate the articulating probe 120 in response to the first control signal.

**FIGs. 8A and 8B** are sectional views of an electrically activated surgical tool. A functional element 141 may be coupled to a distal end of the tool shaft of the surgical tool 140. The surgical tool 140 may include an actuator 142, which may be coupled to or connected to the functional element 141 via mechanical linkage 143.

The functional element 141 may be activated in response to a control signal generated by the HID 210. The actuator 142 of the surgical tool 140 may apply a force to the mechanical linkage 143 in response to the control signal, which may cause first and second claw members or grasper members of the functional element 141 to open and close. The control signal may be an electrical signal, which may be transmitted from the HID 210 to at least one of the controller 115 and the surgical tool 140 via a conductive wire 311.

**FIGs. 9A and 9B** are sectional views of a mechanically activated surgical tool. A functional element 141 may be coupled to a distal end of the tool shaft of the surgical tool 140. The surgical tool 140 may include an actuating cable 118, which may be coupled to or connected to the functional element 141 via mechanical linkage 143.

The functional element 141 may be activated in response to a control signal generated by the HID 210. The control signal may be a mechanical signal, which may be transferred from the HID 210 to the mechanical linkage 143 via the actuating cable 118. The actuating cable 118 may apply a force to the mechanical linkage 143, which may cause first and second claw members or grasper members of the functional element 141 to open and close.

The actuating cable 118 may be further coupled to or connected to a lever 305 of the HID 210. In this manner, a resistance applied to the claw members or grasper members of the functional element 141 may be transferred from the functional element 141 to the lever 305. As such, an operator applying a force to the lever 305 may receive tactile feedback.

**FIG. 10** is a perspective view of a console system. A console system 200 may include a HID 405, a monitor 205, a GUI 215 and an input device or cluster of input devices 440.

The HID 405 may include first and second multi-axis input devices 420, 430, and an input device 410. The first and second multi-axis input device 420, 430 may include one or more features of the multi-function interface described in U.S. Patent No. 7,411,576, issued August 12, 2008, the contents of which is incorporated herein by reference in its entirety.

Each of the first and second multi-axis input devices 420, 430 may include a stylus 425, 435 coupled to an arm of the first and second multi-axis devices 420, 430. The input device 410 may include a displacement member 411 having a proximal and distal ends. A proximal end of the displacement member 411 may be coupled to a ball joint 410a and a distal end of the displacement member 411 may be coupled to a linking member 412. Alternatively or additionally, a universal joint may be used to couple proximal and distal ends of displacement member 411.

The linking member 412 is constructed and arranged to removably couple the first and second styli 425, 435 together. For example, the first stylus 425 may be removably coupled to a first opening of the linking member 412 and the second stylus 435 may be removably coupled to a second opening of the linking member 412.

The HID 405 may be configured to generate the first control signal when a first user interface, such as the first stylus 425 of the first multi-axis input device 420 and a second user interface, such as the second stylus 435 of the second multi-axis input device 430 are coupled together (e.g., via the linking member). As described above, the controller 115 may be constructed and arranged to manipulate (e.g., advance or retract, steer, actuate) the articulating probe 120 in response to the first control signal. For example, the HID 405 may be configured to generate the first control signal when the first user interface of the first multi-axis input device and the second user interface of the second multi-axis input device are synchronously moved while attached to the ends of the linking member 412.

For example, when the first and second user interfaces are coupled, movements of the linking member 412 with respect to the input device 410 may generate control signals for manipulating the articulating probe 120 of the system 100. For example, a movement of the linking member 412 with respect to the input device 410 in Z direction may manipulate and/or advance or retract the articulating probe 120 in a first direction D1 (see for example, the first direction D1 illustrated in FIG 4). A movement of the linking member 412 with respect to the input device 410 in the X direction may manipulate and/or steer the articulating probe 120 in a second direction D2 (see for example, the second direction D2 illustrated in FIG 4). A movement of the linking member 412 with respect to the input device 410 in the Y direction may manipulate and/or steer the articulating probe 120 in a third direction D3 (see for example, the third direction D3 illustrated in FIG 4).

The HID 405 may be further configured to generate the second control signal when the first user interface of the first multi-axis input device 420 and the second user interface of the second multi-axis input device 430 are decoupled. As described above, the controller 115 may be constructed and arranged to manipulate (e.g., advance or retract, steer, actuate) one of the surgical tools 140, 140a-b in response to the second control signal. For example, the HID 405 may be configured to generate the second control signal when the first user interface of the first multi-axis input device 420 and the second user interface of the second multi-axis input device 430 are independently moved.

The console 200 may further include one or more foot pedals 450, which may be configured to generate a control signal. The control signal generated by the one or more foot pedals 450 may be transmitted to the controller 115 and/or the surgical tools 140, 140a-b. A functional element 141, 141a-b of one of the surgical tools 140, 140a-b may be activated in response to the control signal generated by the one or more foot pedals 450.

At the option of the user, the signals generated by the HID 405 and the foot pedals 450 may be reconfigured. For example, the foot pedal 450 may be configured to generate a third control signal where the third control signal may be transmitted to the controller 115 and/or the probe 120.

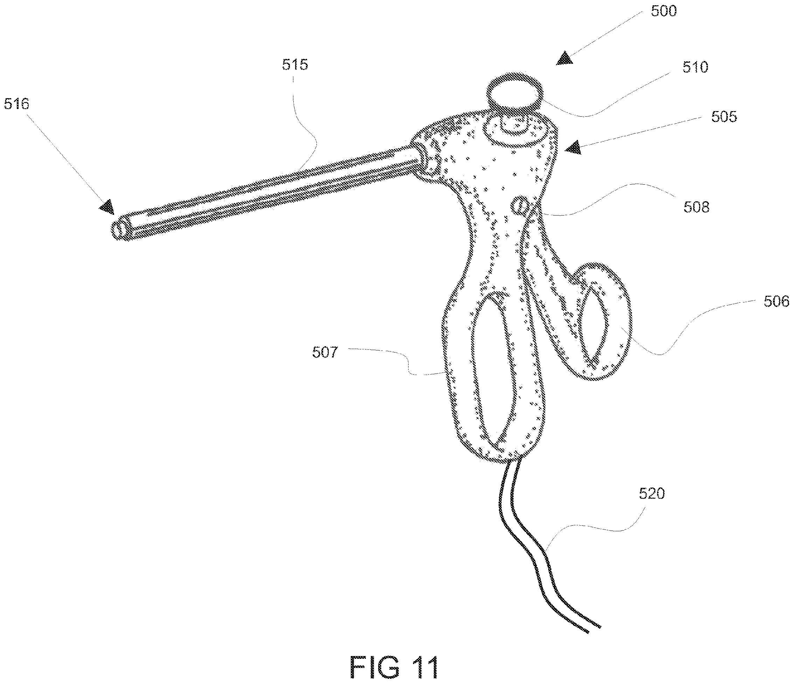

**FIG. 11** is a perspective view of a surgical tool. A surgical tool 500 may include a functional element 516 at distal end of a tool shaft 515. The functional element 516 may include one of the functional elements 141 described above. A proximal end of the tool shaft 515 may be coupled to or connected to a hand-operated lever 505, which may be constructed and arranged to actuate the functional element 516. The hand-operated lever 505 may include first and second hand grips 506, 507, which move relative to one another about an axis 508.

The surgical tool 500 may further include a joystick 510. The joystick 510 may be integral with the hand operated lever 505; however, in other embodiments the joystick 510 may be coupled to or connected to the hand operated lever 505. For example, in some embodiments, the joystick 510 is removably coupled to the second surgical tool, such as by way of a mechanical snap fit, clip, thumbscrew or magnetic fastening elements. In some embodiments, the joystick 510 is affixed to the hand operated lever 505 of the surgical tool 500 with an adhesive or other non-removable fastening element.

The joystick 510 may be configured to generate a control signal, which may be transmitted (via a conductive wire or cable 520) to the console 200 and/or the controller 115 of the systems 100 described above. However, the control signal may be wirelessly transmitted to the console 200 and/or the controller 115. In this manner, an operator, such as a surgeon or medical professional, can command the systems 200 to manipulate the articulating probe 120 from a signal interfacing device.

While the present invention has been particularly shown and described above with reference to exemplary embodiments thereof, it will be understood by those of ordinary skill in the art, that various changes in form and detail can be made without departing from the scope of the present invention described and defined by the following claims.

## Claims

1. A console system (200) comprising a system (100), the system (100) comprising:
an articulating probe (120);
a surgical tool (140, 140a-b, 500);
a controller (115) constructed and arranged to manipulate the articulating probe (120) and the surgical tool (140, 140a-b, 500); and
a single human interface device (HID) (405) configured to generate a first control signal and a second control signal,
wherein the articulating probe (120) is manipulated in response to the first control signal and the surgical tool (140, 140a-b, 500) is manipulated in response to the second control signal,
the human interface device (HID) (405) includes first and second multi-axis input devices (420, 430), and an input device (410), **characterised in that**
each of the first and second multi-axis input devices (420, 430) include a stylus (425, 435) coupled to an arm of the first and second multi-axis devices (420, 430), and further wherein
the input device (410) includes a displacement member (411) having a proximal and distal ends, wherein
a proximal end of the displacement member (411) coupled to a ball joint (410a) and a distal end of the displacement member (411) be coupled to a linking member (412) and wherein the linking member (412) is constructed and arranged to removably couple the first and second styli (425, 435) together.

2. The console system (200) of claim 1, wherein the articulating probe (120) includes a light source (165) at a distal end (135) of the articulating probe (120), wherein the light source (165) is activated in response to a third control signal generated by the HID (210), the third control signal being an electrical signal.

3. The console system (200) of at least one of the preceding claims, wherein the third control signal is transmitted from the HID (210) to the electromechanical feeder system via a conductive wire (520).

4. The console system (200) of at least one of the preceding claims, wherein the articulating probe (120) includes an image capture device (160) at a distal end (135) of the articulating probe (120), wherein the image capture device (160) is activated in response to a fourth control signal generated by the HID (210), the fourth control signal being transmitted from the HID (210) to the electromechanical feeder system via a conductive wire (520).

5. The console system (200) of at least one of the preceding claims, wherein the image capture device (160) transmits an image signal to the HID (210).

6. The console system (200) of at least one of the preceding claims, wherein said controller (115) is constructed and arranged to advance or retract the surgical tool (140, 140a-b, 500) in response to the second control signal.

7. The console system (200) of at least one of the preceding claims, wherein said controller (115) is constructed and arranged to steer the articulating probe (120) in response to the first control signal.

8. The console system (200) of at least one of the preceding claims, wherein the surgical tool (140, 140a-b, 500) includes a tool shaft (515) having an articulation region and/or a functional element (141, 141a-b) coupled to a distal end (135) of the tool shaft (515), wherein the functional element (141, 141a-b) includes one selected from the group consisting of: a grasper, a claw, a cutter, a knife, an ablator, a cauterizer, a drug delivery apparatus, a radiation source, an EKG electrode, a pressure sensor, a blood sensor, a camera, a light source (165), a snare, a basket, a balloon, a clamp, a magnet, a heating element and a cryogenic element.

9. The console system (200) of at least one of the preceding claims, wherein the articulation region of the tool shaft (515) includes at least two segment links.

10. The console system (200) of at least one of the preceding claims, wherein said controller (115) is constructed and arranged to advance or retract the distal end (135) of the tool shaft (515) with respect to a distal end (135) of the articulating probe (120).

11. The console system (200) of at least one of the preceding claims 8-10, wherein the surgical tool (140, 140a-b, 500) includes an actuator (116, 116a-b, 350, 142), the actuator coupled to the functional element (141, 141a-b) via mechanical linkage (143).

12. The console system (200) of at least one of the preceding claims 8-11, wherein the functional element (141, 141a-b) is activated in response to a other control signal generated by the HID (210).

13. The console system (200) of at least one of the preceding claims, wherein said controller (115) simultaneously or sequentially manipulates the articulating probe (120) and the surgical tool (140, 140a-b, 500).

## Patentansprüche

1. Konsolensystem (200) mit einem System (100), wobei das System (100) umfasst:
eine Gelenksonde (120);
ein chirurgisches Werkzeug (140, 140a-b, 500);
eine Steuereinheit (115), die konstruiert und angeordnet ist, um die Gelenksonde (120) und das chirurgische Werkzeug (140, 140a-b, 500) zu betätigen; und
ein einzelnes Human Interface Device (HID) (405), das so konfiguriert ist, dass es ein erstes Steuersignal und ein zweites Steuersignal erzeugt,
wobei die Gelenksonde (120) in Reaktion auf das erste Steuersignal betätigt wird und das chirurgische Werkzeug (140, 140a-b, 500) in Reaktion auf das zweite Steuersignal betätigt wird,
und wobei das Human Interface Device (HID) (405) eine erste und eine zweite mehrachsige Eingabeeinrichtung (420, 430) und eine Eingabeeinrichtung (410) umfasst,
**dadurch gekennzeichnet, dass**
jede der ersten und zweiten mehrachsigen Eingabeeinrichtungen (420, 430) einen Stift (425, 435) enthält, der mit einem Arm der ersten und zweiten mehrachsigen Geräte (420, 430) gekoppelt ist, und weiter wobei die Eingabeeinrichtung (410) ein Verschiebungselement (411) mit einem proximalen und einem distalen Ende aufweist, wobei
ein proximales Ende des Verschiebungselements (411), mit einem Kugelgelenk (410a) gekoppelt ist und ein distales Ende des Verschiebungselements (411) mit einem Verbindungselement (412) gekoppelt ist und wobei das Verbindungselement (412) konstruiert und angeordnet ist, um den ersten und den zweiten Stift (425, 435) lösbar miteinander zu verbinden.

2. Konsolensystem (200) nach Anspruch 1, wobei die Gelenksonde (120) eine Lichtquelle (165) an einem distalen Ende (135) der Gelenksonde (120) aufweist, wobei die Lichtquelle (165) als Reaktion auf ein drittes Steuersignal aktiviert wird, das durch das HID (210) erzeugt wird, wobei das dritte Steuersignal ein elektrisches Signal ist.

3. Konsolensystem (200) nach mindestens einem der vorhergehenden Ansprüche, wobei das dritte Steuersignal vom HID (210) über einen leitenden Draht (520) an das elektromechanische Zuführsystem übertragen wird.

4. Konsolensystem (200) nach mindestens einem der vorhergehenden Ansprüche, wobei die Gelenksonde (120) eine Bildaufnahmeeinrichtung (160) an einem distalen Ende (135) der Gelenksonde (120) aufweist, wobei die Bildaufnahmeeinrichtung (160) in Reaktion auf ein viertes Steuersignal aktiviert wird, das durch das HID (210) erzeugt wird, wobei das vierte Steuersignal vom HID (210) über einen leitenden Draht (520) an das elektromechanische Zuführsystem übertragen wird.

5. Konsolensystem (200) nach mindestens einem der vorhergehenden Ansprüche, wobei die Bildaufnahmeeinrichtung (160) ein Bildsignal zum HID (210) überträgt.

6. Konsolensystem (200) nach wenigstens einem der vorhergehenden Ansprüche, wobei die Steuerung (115) so konstruiert und angeordnet ist, dass sie das chirurgische Werkzeug (140, 140a-b, 500) in Reaktion auf das zweite Steuersignal vor- bzw. zurückbewegt.

7. Konsolensystem (200) nach wenigstens einem der vorhergehenden Ansprüche, wobei die Steuerung (115) so konstruiert und angeordnet ist, dass sie die Gelenksonde (120) in Reaktion auf das erste Steuersignal steuert.

8. Konsolensystem (200) nach wenigstens einem der vorhergehenden Ansprüche, wobei das chirurgische Werkzeug (140, 140a-b, 500) einen Werkzeugschaft (515) umfasst, der einen Gelenkbereich und/oder ein Funktionselement (141, 141a-b) aufweist, das mit einem distalen Ende (135) des Werkzeugschaftes (515) gekoppelt ist, wobei das Funktionselement (141, 141a-b) eines enthält, ausgewählt aus der Gruppe bestehend aus: Einem Greifer, einer Kralle, einem Cutter, einem Messer, einem Ablator, einem Kauter, einer Medikamentenabgabeeinrichtung, einer Strahlungsquelle, einer EKG-Elektrode, einem Drucksensor, einem Blutsensor, einer Kamera, einer Lichtquelle (165), einer Schlinge, einem Korb, einem Ballon, einer Klemme, einem Magneten, einem Heizelement und einem kryogenen Element.

9. Konsolensystem (200) nach wenigstens einem der vorhergehenden Ansprüche, wobei der Gelenkbereich des Werkzeugschaftes (515) mindestens zwei Segmentglieder aufweist.

10. Konsolensystem (200) nach wenigstens einem der vorhergehenden Ansprüche, wobei die Steuerung (115) so konstruiert und angeordnet ist, dass sie das distale Ende (135) des Werkzeugschaftes (515) in Bezug auf ein distales Ende (135) der Gelenksonde (120) vor- oder zurückzieht.

11. Konsolensystem (200) nach wenigstens einem der vorhergehenden Ansprüche 8 - 10, wobei das chirurgische Werkzeug (140, 140a-b, 500) einen Aktuator (116, 116a-b, 350, 142) umfasst, wobei der Aktuator über eine mechanische Verbindung (143) mit dem Funktionselement (141, 141a-b) gekoppelt ist.

12. Konsolensystem (200) nach wenigstens einem der vorhergehenden Ansprüche 8 - 11, wobei das Funktionselement (141, 141a-b) in Reaktion auf ein anderes, durch das HID (210) erzeugtes Steuersignal aktiviert wird.

13. Konsolensystem (200) nach wenigstens einem der vorhergehenden Ansprüche, wobei die Steuerung (115) gleichzeitig oder sequentiell die Gelenksonde (120) und das chirurgische Werkzeug (140, 140a-b, 500) betätigt.

## Revendications

1. Système de console (200) comprenant un système (100), le système (100) comprenant :
une sonde articulée (120) ;
un outil chirurgical (140, 140a-b, 500) ;
un dispositif de commande (115) construit et agencé pour manipuler la sonde articulée (120) et l'outil chirurgical (140, 140a-b, 500) ; et
un unique dispositif d'interface humaine (HID) (405) configuré pour produire un premier signal de commande et un deuxième signal de commande,
dans lequel la sonde articulée (120) est manipulée en réponse au premier signal de commande et l'outil chirurgical (140, 140a-b, 500) est manipulé en réponse au deuxième signal de commande,
le dispositif d'interface humaine (HID) (405) inclut des premier et second dispositifs d'entrée multiaxiaux (420, 430) et un dispositif d'entrée (410),
**caractérisé en ce que**
chacun des premier et second dispositifs d'entrée multiaxiaux (420, 430) incluent un stylet (425, 435) couplé à un bras des premier et second dispositifs d'entrée multiaxiaux (420, 430), et en outre dans lequel
le dispositif d'entrée (410) inclut un élément de déplacement (411) présentant une extrémité proximale et distale, dans lequel
une extrémité proximale de l'élément de déplacement (411) couplée à un joint sphérique (410a) et une extrémité distale de l'élément de déplacement (411) est couplée à un élément de liaison (412),
et dans lequel l'élément de liaison (412) est construit et agencé pour coupler de manière amovible les premier et second stylets (425, 435) ensemble.

2. Système de console (200) selon la revendication 1, dans lequel la sonde articulée (120) inclut une source de lumière (165) à une extrémité distale (135) de la sonde articulée (120), dans lequel la source de lumière (165) est activée en réponse à un troisième signal de commande produit par le HID (210), le troisième signal de commande étant un signal électrique.

3. Système de console (200) selon au moins l'une des revendications précédentes, dans lequel le troisième signal de commande est émis depuis le HID (210) vers le système d'alimentation électromécanique via un fil conducteur (520).

4. Système de console (200) selon au moins l'une des revendications précédentes, dans lequel la sonde articulée (120) inclut un dispositif de capture d'image (160) à une extrémité distale (135) de la sonde articulée (120), dans lequel le dispositif de capture d'image (160) est activé en réponse à un quatrième signal de commande produit par le HID (210), le quatrième signal de commande étant émis depuis le HID (210) vers le système d'alimentation électromécanique via un fil conducteur (520).

5. Système de console (200) selon au moins l'une des revendications précédentes, dans lequel le dispositif de capture d'image (160) émet un signal d'image vers le HID (210).

6. Système de console (200) selon au moins l'une des revendications précédentes, dans lequel ledit dispositif de commande (115) est construit et agencé pour avancer ou rétracter l'outil chirurgical (140, 140a-b, 500) en réponse au deuxième signal de commande.

7. Système de console (200) selon au moins l'une des revendications précédentes, dans lequel ledit dispositif de commande (115) est construit et agencé pour diriger la sonde articulée (120) en réponse au premier signal de commande.

8. Système de console (200) selon au moins l'une des revendications précédentes, dans lequel l'outil chirurgical (140, 140a-b, 500) inclut un arbre d'outil (515) présentant une région d'articulation et/ou un élément fonctionnel (141, 141a-b) couplé à une extrémité distale (135) de l'arbre d'outil (515), dans lequel l'élément fonctionnel (141, 141a-b) inclut l'un sélectionné dans le groupe consistant en : un préhenseur, une pince, une lame, un couteau, un ablateur, un cautérisateur, un appareil d'administration de médicament, une source de rayonnement, une électrode ECG, un capteur de pression, un capteur sanguin, une caméra, une source de lumière (165), une anse, un panier, un ballon, un clamp, un aimant, un élément de chauffage et un élément cryogénique.

9. Système de console (200) selon au moins l'une des revendications précédentes, dans lequel la région d'articulation de l'arbre d'outil (515) inclut au moins deux liaisons de segment.

10. Système de console (200) selon au moins l'une des revendications précédentes, dans lequel ledit dispositif de commande (115) est construit et agencé pour avancer ou rétracter l'extrémité distale (135) de l'arbre d'outil (515) par rapport à une extrémité distale (135) de la sonde articulée (120).

11. Système de console (200) selon au moins l'une des revendications précédentes 8-10, dans lequel l'outil chirurgical (140, 140a-b, 500) inclut un actionneur (116, 116a-b, 350, 142), l'actionneur couplé à l'élément fonctionnel (141, 141a-b) via une liaison mécanique (143).

12. Système de console (200) selon au moins l'une des revendications précédentes 8-11, dans lequel l'élément fonctionnel (141, 141a-b) est activé en réponse à un autre signal de commande produit par le HID (210).

13. Système de console (200) selon au moins l'une des revendications précédentes, dans lequel ledit dispositif de commande (115) manipule simultanément ou séquentiellement la sonde articulée (120) et l'outil chirurgical (140, 140a-b, 500).
